# EUROPEAN PATENT SPECIFICATION

(11) **EP 3 220 911 B1**
(45) Date of publication and mention of the grant of the patent: **07.11.2018**
(21) Application number: 15808807.0
(22) Date of filing: 16.11.2015
(51) Int. Cl.: A61K 31/50, A61K 9/08, A61P 1/16

(54) **LEVOSIMENDAN FOR USE AS A LIVER PROTECTOR**
LEVOSIMENDAN ZUR VERWENDUNG ALS LEBERSCHUTZ
UTILISATION DU LÉVOSIMENDAN COMME PROTECTEUR HÉPATIQUE

(30) Priority: 17.11.2014 IT RM20140672
(43) Date of publication of application: 27.09.2017
(73) Proprietor: Universitá Degli Studi Di Salerno, 84084 Fisciano (SA) (IT)
(72) Inventor: PIAZZA, Ornella, I-80129 Napoli (IT); SCARPATI, Giuliana, I-80069 Vico Equense (NA) (IT)
(74) Representative: Germinario, Claudio
(86) International application number: PCT/IB2015/058845
(87) International publication number: WO 2016/079652

(56) References cited:
- Anonymous: "Summary of product characteristics - Simdax 2.5 mg/ml concentrate for solution for infusion", Orion Pharma , 11 June 2010 (2010-06-11), XP002740013, Retrieved from the Internet: URL:http://www.simdax.com/SimdaxCom_Global /SIMDAX%20SPC.pdf [retrieved on 2015-05-26]
- PUTTONEN JAAKKO ET AL: "Pharmacokinetics of intravenous levosimendan and its metabolites in subjects with hepatic impairment.", JOURNAL OF CLINICAL PHARMACOLOGY, vol. 48, no. 4, April 2008 (2008-04), pages 445-454, XP002740014, ISSN: 0091-2700
- GUERRERO ORRIACH J L ET AL: "Preoperative levosimendan. A new way for organoprotection", CURRENT PHARMACEUTICAL DESIGN, BENTHAM SCIENCE PUBLISHERS, NL, vol. 20, no. 34, 1 January 2014 (2014-01-01), pages 5476-5483, XP009184461, ISSN: 1381-6128
- Civan: "Cirrhosis", Merck Manual Merck Manual Professional Version, 2013, pages 1-9, XP002753662, Retrieved from the Internet: URL:https://www.merckmanuals.com/professio nal/hepatic-and-biliary-disorders/fibrosis -and-cirrhosis/cirrhosis [retrieved on 2016-02-01]

## Description

The present invention relates to the compound ({4 - [(4'R)-4-Methyl-6-oxo-1,4,5,6-tetrahydropyridazin-3-yl] phenyl} hydrazono) propandinitrile (levosimendan) for use in the treatment of liver cirrhosis, in particular with an inhibitory action limiting the development of liver fibrosis and brain metabolic alterations correlated to cirrhosis.

### State of art

Levosimendan ({4-[(4'R)-4-Methyl-6-oxo-1,4,5,6-tetrahydropyridazin-3-yl] phenyl} hydrazono) is a cardioactive drug with anti-ischemic properties, since it acts onto the opening of the mitochondrial ATP-sensitive potassium channels in the cardiac miocells. It is used in the emergency therapy field, in the treatment of heart failure in decompensated phase.

Liver cirrhosis is a chronic and widespread process characterized by fibrosis and by the transformation of the normal liver architecture in structurally abnormal nodules.

Liver cirrhosis is associated to a state of portal hypertension which is determined by an increase in the intrahepatic resistances and the portal blood flow. The increase in the resistances to the portal flow is caused by mechanical and haemodynamic factors. The mechanical factors are fibrosis, the parenchymatous nodules formed by regenerated hepatocytes, the microthromboses, the capillarization of the sinusoids due to the collagen deposition by the stellate cells (Ito cells) stimulated by cytokines and toxic substances. The potentially reversible hemodynamic factors are determined by an unbalance between factors of vasodilators and vasoconstrictors with prevalence of the latter (increased production of endothelin 1, PGH2, thromboxane A2 and NO deficit due to decreased activity of eNOS and increased degradation). When hepatopathy becomes more serious, the vasoconstriction gets more serious with consequent ischaemia of essential organs, leading to the onset of complications such as hepatorenal syndrome, lung hypertension up to Multiple Organ Failure (MOF).

Liver encephalopathy is a complication of liver cirrhosis, the pathogenesis thereof is still nowadays a much discussed and not yet explained matter.

For liver cirrhosis and brain complications thereof no valid pharmacological treatment was available. Other drugs were tested in past with very poor results in the cirrhotic patient: for example in the NAFLD (nonalcoholic fatty liver disease) cirrhosis one tried to administer Metformin 2 g/die (a drug which sensitizes cells to insulin, by reducing the insulin-resistance) with marginal results on the levels of transaminases and no evident effect during the histological examination of the liver biopsies (Shields WW et AI. Therap Adv Gastroenterol 2009;2:157-63).

Corticosteroids, another class of drugs used in the treatment of alcoholic cirrhosis, have a controversial use considering that they increase the risk of serious infections. Therefore it was proposed to replace steroids by IL1 inhibitors, important mediator of inflammation. IL-1Ra, an antagonist of IL-1β (Anakinra) limits the increase in ALT in rats to which alcoholic steatosis was induced but the therapeutic potential thereof and tolerability have to be better defined (Petrasek J et Al J Clin Invest. Oct 1, 2012; 122(10): 3476-3489), since the above-mentioned experiments do not consider the case of nodular cirrhosis and the effects on the fully manifest cirrhosis, but on steatosis only.

J. Puttonen et al. (J. Clin. Pharmacol. 2008; 48:445-454) discloses the intravenous administration of levosimendan for treating acute heart failure in subjects with moderate hepatic impairment.

In the light of what just illustrated, thus in the state of art the need was felt for having available an effective pharmacological treatment.

Now it has been surprisingly found that levosimendan can be used as hepatoprotector drug and such use is not provided at all nor derivable from the state of art.

The subject of the present invention is the compound levosimendan for use according to claim 1. Additional implementations are described in the depending claims.

### Brief description of the drawings

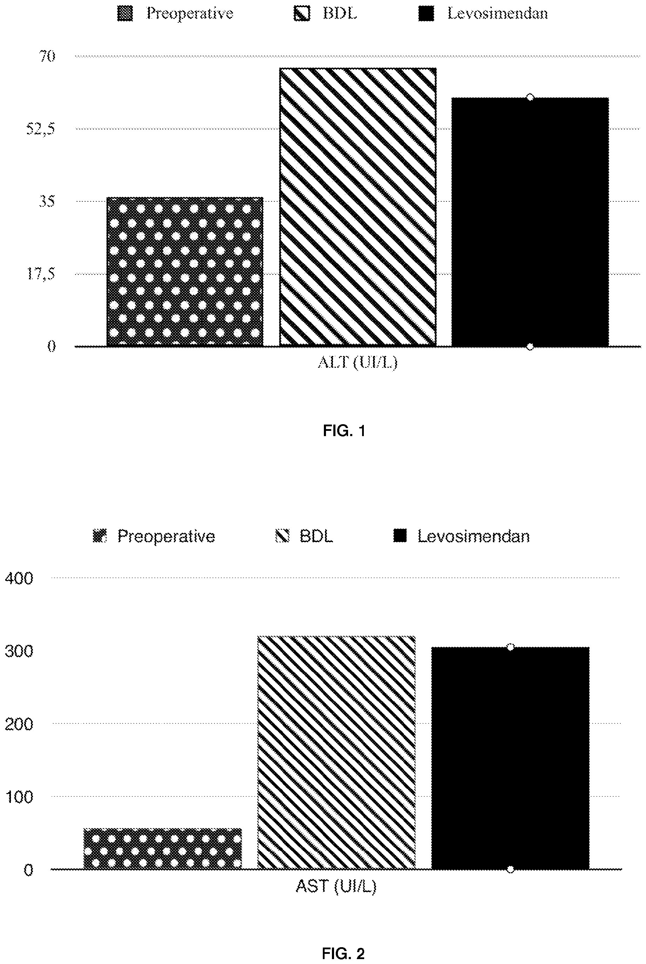

Two figures are enclosed to the present description wherein
Figure 1 shows how the average plasmatic values of alanine amino transferase (ALT) from blood samples extracted from the caudal vein increase two weeks after the surgical operation (BDL column) with respect to what observed in the same animals at the beginning of the protocol (preoperative column); by observing the third column, it is to be noted that 4 days after treatment (Levosimendan column) the ALT values decrease significantly (cirrhotic rat average value ALT 67,166 UI/L versus average value of cirrhotic rat treated with levosimendan ALT 60 UI/L) and
Figure 2 highlights how the average plasmatic values of aspartate transaminase (AST) from blood samples extracted from the caudal vein increase two weeks after the surgical operation (BDL column) with respect to what observed in the same animals at the beginning of the protocol (preoperative column); 4 days after treatment (Levosimendan column) they decrease significantly (cirrhotic rat average value AST 320 Ul/L versus average value of cirrhotic rat treated with levosimendan: AST 304 UI/L).

### Description of the invention

Liver cirrhosis is a chronic and widespread process characterized by fibrosis and by the transformation of the liver normal architecture in structurally abnormal nodules.

Hepatic encephalopathy is a complication of liver cirrhosis; during hepatic encephalopathy the normal brain metabolism is altered with a mechanism not fully explained up to now.

The inventors of the present invention evaluated the effect of levosimendan ({4-[(4'R)-4-Methyl-6-oxo-1,4,5,6-tetrahydropyridazin-3-yl] phenyl} hydrazono) propandinitrile in a murine experimental model of liver failure induced by binding the biliary duct. The chronic liver failure in rat can be experimentally induced through the double binding of the common biliary duct. Such procedure causes morphological changes in the hepatic tissue superimposable to hepatic fibrosis and cirrhosis in man. The duct binding surgical procedure is considerably more advantageous with respect to other experimental procedures such as the intraperitoneal injection of carbon tetrachloride (CCl4) or dimethylinitrosamine (DMN), since the individual response of each animal to exposure to said hepatotoxic substances is variable, mortality is high, and at last such substances induce fibrosis or hepatic cirrhosis in considerably longer time than the surgical procedure.

50 Wistar male rats with average weight of 200 grams were divided into 4 groups:
Group I: control.
Group II: rats with hepatic cirrhosis induced by binding of the biliary duct (BLD)
Group III: rats with hepatic cirrhosis induced by binding the biliary duct and subsequent administration of levosimendan.
Group IV: rats treated with levosimendan but without induction of hepatic disease.

In order to monitor the experimentally determined fibrogenesis, bilirubin concentrations and the serum activities of aspartate transaminase (AST) e alanine amino transferase (ALT) were measured from blood samples extracted from the caudal vein at the beginning of the protocol, 2 weeks after the surgical intervention and 4 days after treatment. The rats were sacrificed 14 days after binding the biliary duct.

In rats the normal hepatic activity is expressed by the average values:
- Bilirubin: 0.14 ± 0.05 mg/dl
- AST 53.49 ± 17.31 U/I
- ALT 28.18 ± 7.84 U/I

In all groups brain and hepatic microdialysis was performed. Hepatic histological examination was performed after having sacrificed the experiment animal and after having classified the result according to Scheuer system in degrees from 0 to 3 (no fibrosis, low fibrosis, moderated fibrosis, serious fibrosis). Levosimendan (Simdax, Orion Pharma) diluted in 10 ml of 0.5% dextrose was administered through single intraperitoneal injection with a dosage of 12 micrograms/Kg.

Bilirubin, AST and ALT in the group of cirrhotic rats (BDL) were sensibly higher than in the control group, however, the serum levels of ALT, AST and bilirubin reduced significantly in the group treated with levosimendan (cirrhotic rat average value: bilirubin 6.416 mg/dl, ALT 67,166 UI/L, AST 320 UI/L versus average value of cirrhotic rat treated with levosimendan: bilirubin 3.9 mg/dl, ALT 60 UI/L, AST 304 UI/L). The results of hepatic microdialysis showed an altered metabolic activity in the rats of group BLD, which tended to normalize in the group BLD+levosimendan. At the brain level, the glucose concentration in the microdialysate decreased in cirrhotic rats, with concomitant increase in lactate: this pathological framework improved in the cirrhotic rat treated with levosimendan.

During the histological examination of liver, in the control group preserved and intact hepatic parenchyme (stage 0) was found. In all rats of (not treated) group BDL micronodular cirrhosis and serious fibrosis (stage 3) were found. In group BDL +levosimendan the damage was significantly lower than in the not treated cirrhotic rats: in fact, in all rats, the portal tracts were oedematous and affected by biliary fibrosis, with irregular contours of the portal spaces, but the classification was included in stages 1-2. Even such histological result confirms the hepatoprotecting capabilities of levosimendan being expressed with a direct anti-fibrotic action.

All above demonstrates that the administration of levosimendan reduces the formation of fibrotic nodules in the liver and thus the concentration of bilirubin in blood. The administration of levosimendan reduces the suffering of liver wherein cirrhosis was induced and this is demonstrated by the reduced levels of AST and ALT. What illustrated above further demonstrates that levosimendan improves the metabolism and thus the brain functionality during liver cirrhosis, as highlighted by the decrease in the lactate levels in microdyalisate.

Additional subjects of the present invention are pharmaceutical compositions comprising the compound levosimendan for use in the treatment of liver cirrhosis and pharmaceutically tolerable additives.

Advantageously a composition according to the present invention comprises, in addition to the active principle, povidone, citric acid and ethanol; for the dilution advantageously a preferably 5% glucose solution is used. The administration route is intravenous only. Advantageously the administration is an infusion in slow (in about 10 minutes) intravenous bolus of 6 - 12 micrograms/kg, preferably 8-10 mcg/kg.

## Claims

1. A compound ({4-[(4'R)-4-Methyl-6-oxo-1,4,5,6-tetrahydropyridazin-3-yl] phenyl} hydrazono) for use in the treatment of liver cirrhosis.

2. The compound according to claim 1 for use in the treatment of liver cirrhosis in order to limit the progression of liver fibrosis.

3. The compound according to claim 1 for use in the treatment of liver cirrhosis accompanied by hepatic encephalopathy to improve altered cerebral metabolism.

4. Pharmaceutical compositions for use in the treatment of liver cirrhosis comprising a therapeutically effective amount of the compound ({4-[(4'R)-4-Methyl-6-oxo-1,4,5,6-tetrahydropyridazin-3-yl] phenyl} hydrazono) and pharmaceutically tolerable additives.

5. The composition according to claim 4 for use in the treatment of liver cirrhosis in order to limit the progression of liver fibrosis.

6. The composition according to claim 4 for use in the treatment of liver cirrhosis accompanied by hepatic encephalopathy to improve altered cerebral metabolism.

## Patentansprüche

1. Verbindung ({4-[(4'R)-4-Methyl-6-oxo-1,4,5,6-tetrahydropyridazin-3-yl]phenyl}hydrazono) zur Verwendung bei der Behandlung von Leberzirrhose.

2. Verbindung gemäß Anspruch 1 zur Verwendung bei der Behandlung von Leberzirrhose, um den Fortschritt von Leberzirrhose zu begrenzen.

3. Verbindung gemäß Anspruch 1 zur Verwendung bei der Behandlung von Leberzirrhose, die begleitet durch hepatische Enzephalopathie wird, um einen veränderten cerebralen Metabolismus zu verbessern.

4. Pharmazeutische Zusammensetzung zur Verwendung bei der Behandlung von Leberzirrhose, die umfasst: eine therapeutisch wirksame Menge der Verbindung ({4-[(4'R)-4-Methyl-6-oxo-1,4,5,6-tetrahydropyridazin-3-yl]phenyl}hydrazono) und pharmazeutisch tolerierbare Zusatzstoffe.

5. Zusammensetzung gemäß Anspruch 4 zur Verwendung bei der Behandlung von Leberzirrhose, um den Fortschritt von Leberzirrhose zu begrenzen.

6. Zusammensetzung gemäß Anspruch 4 zur Verwendung bei der Behandlung von Leberzirrhose, die begleitet durch hepatische Enzephalopathie wird, um einen geänderten zerebralen Metabolismus zu verbessern.

## Revendications

1. Composé de ({4-[(4'R)-4-méthyl-6-oxo-1,4,5,6-tétra-hydropyridazin-3-yl]phényl}hydrazono) pour une utilisation dans le traitement de la cirrhose du foie.

2. Composé selon la revendication 1 pour son utilisation dans le traitement de la cirrhose du foie afin de limiter la progression de la fibrose du foie.

3. Composé selon la revendication 1 pour son utilisation dans le traitement d'une cirrhose du foie s'accompagnant d'encéphalopathie hépatique pour améliorer le métabolisme cérébral altéré.

4. Compositions pharmaceutiques pour une utilisation dans le traitement de la cirrhose du foie comprenant une quantité thérapeutiquement efficace du composé de ({4-[(4'R)-4-méthyl-6-oxo-1,4,5,6-tétrahydropyridazin-3-yl]phényl}-hydrazono) et d'additifs pharmaceutiquement tolérables.

5. Composition selon la revendication 4 pour son utilisation dans le traitement de la cirrhose du foie afin de limiter la progression de la fibrose du foie.

6. Composition selon la revendication 4 pour son utilisation dans le traitement d'une cirrhose du foie s'accompagnant d'encéphalopathie hépatique pour améliorer le métabolisme cérébral altéré.
